# EUROPÄISCHE PATENTSCHRIFT

(11) **EP 1 715 032 B1**
(45) Veröffentlichungstag und Bekanntmachung des Hinweises auf die Patenterteilung: **07.12.2011**
(21) Anmeldenummer: 06006218.9
(22) Anmeldetag: 25.03.2006
(51) Int. Cl.: C12M 1/40

(54) **Verfahren zur fermentativen Herstellung von Duft-und/oder Aromastoffen**
Fermentation process for preparing perfuming and flavouring agents
Procédé de fermentation pour la préparation d'agents parfumants et aromatisants

(30) Priorität: 20.04.2005 DE 102005018256
(43) Veröffentlichungstag der Anmeldung: 25.10.2006
(73) Patentinhaber: DECHEMA Gesellschaft für Chemische Technik und Biotechnologie e.V., 60486 Frankfurt (DE)
(72) Erfinder: Etschmann, Marlene, Dr., 60316 Frankfurt am Main (DE); Schrader, Jens, Dr., 60316 Frankfurt am Main (DE); Sell, Dieter, Dr., 61206 Wöllstadt (DE)
(74) Vertreter: Meyer-Dulheuer, Karl-Hermann

(56) Entgegenhaltungen:
- EP-A- 0 164 608
- EP-A- 1 158 042
- WO-A-00/73485
- WO-A-91/06552
- WO-A-03/048335
- DE-A1- 3 236 496
- DE-A1- 10 022 447
- US-A- 4 517 298
- US-A- 4 865 973
- US-B1- 6 812 007

## Beschreibung

Gegenstand der Erfindung ist ein Verfahren zur fermentativen Herstellung von 2-Phenylethanol und/oder 2-Phenyletylacetat aus L-Phenylalanin als Duft- und/oder Aromastoffen in einem Bioreaktor.

Aus der europäischen Patentanmeldung EP 1 158 042 A2 ist bereits ein Verfahren zur biotechnologischen Erzeugung von Duft- und/oder Aromastoffen bekannt, die in einer Flüssigphase eines Bioreaktors durch mikroorganismen- und/oder enzyminduzierte Stoffumwandlungsprozesse erzeugt und mittels einer angeschlossenen Pervaporationseinrichtung isoliert werden. Dabei werden die Zielprodukte kontinuierlich oder sequenziell aus der Flüssigphase abgetrennt. Das Verfahren wird bei Prozesstemperaturen >25°C unter Verwendung thermotoleranter und/oder thermophiler Mikroorganismen und/oder Enzymen durchgeführt. Befriedigende Ausbeuten werden bei diesem technisch aufwendigen Verfahren jedoch nicht erzielt, insbesondere nicht für relativ polare Aromastoffe wie das im vorliegenden Fall unter anderem als Zielprodukt definierte 2-Phenylethanol.

Aus der US-Patentschrift 4 865 973 ist weiterhin ein Verfahren zur Herstellung eines Fermentationsproduktes bekannt, bei dem ein Mikroorganismus, der fähig ist, das gewünschte Produkt herzustellen, in einem wässrigen Fermentationsmedium kultiviert und das Produkt in situ aus dem Medium entfernt wird, indem dieses mit einem Exktraktionsmittel, das mit dem wässrigen Medium nicht mischbar ist, in Kontakt gebracht wird. Als derartige Extraktionsmittel werden gesättigte oder ungesättigte aliphatische Alkohole mit mehr als 12 Kohlenstoffatomen, ungesättigte aliphatische Säuren oder Ester mit mehr als 12 Kohlenstoffatomen sowie aliphatische Ketone und Aldehyde mit 12 oder mehr Kohlenstoffatomen verwendet. Nach diesem bekannten Verfahren werden zum Beispiel Penicillin, Zitronensäure und Polysaccharide gewonnen.

Fermentativ aus natürlichen Rohstoffen hergestellten Produkten wird heutzutage gegenüber chemisch hergestellten Produkten häufig der Vorzug bei der Herstellung von Nahrungs- und Körperpflegemitteln gegeben. Hierzu gehören auch die Duft- und Aromastoffe. Es stellt sich deshalb zunehmend die Aufgabe, für die Herstellung dieser Produkte technisch einsetzbare und ausbeutereiche Verfahren zu entwickeln. Zu den besonders begehrten Duft- und Aromastoffen gehören auch 2-Phenylethanol und 2-Phenylethylacetat.

Die biotechnologische Herstellung von 2-Phenylethanol, welches einen rosenähnlichen Duft aufweist, ist bereits in Appl. Microbiol. Biotechnol. (2002) 59: 1 - 8 beschrieben worden. Weitere Verbesserungen dieses Verfahrens, das auf der Biokonversion von L-Phenyalanin mit Hefen in melassehaltigen Nährmedien beruht, sind in Biotechnology Letters 25: 531 - 536, 2003 und im Journal of Molecular Catalysis B: Enzymatic 29 (2004) 187 - 193 beschrieben.

Gegenstand der Erfindung ist nun ein Verfahren zur fermentativen Herstellung von 2-Phenylethanol und/oder 2-Phenylethylacetat aus L-Phenylalanin in einem Bioreaktor, bei dem man einen Mikroorganismus, der die gewünschten Duft- oder Aromastoffe bilden kann, in einem Zweiphasensystem, bestehend aus einem wässrigen Nährmedium und aus einem mit Wasser nicht mischbaren Extraktionsmittel, welches einen hohen Verteilungskoeffizienten für den entstehenden Duft- oder Aromastoff aufweist und ihn in situ aus dem wässrigen Nährmedium entfernt, auf die als Stickstoffquelle dienende L-Phenylalanin einwirken lässt. Dabei wird als Extraktionsmittel Polypropylenglykol mit einem Molekulargewicht >1.000 und L-Phenylalanin in einer ihre Löslichkeitsgrenze überschreitenden Menge eingesetzt.

Besonders bevorzugt für dieses Verfahren ist Polypropylenglykol mit einem Molekulargewicht von 1.200 bis 1.800.

Für das beschriebene Verfahren können die verschiedensten Aminosäuren als Ausgangsstoffe verwendet werden. Besonders attraktive Reaktionsprodukte 70487 in synthetischem Medium, jeweils mit Polypropylenglykol 1.200, lassen sich herstellen, wenn L-Phenylalanin als Ausgangsstoffe eingesetzt werden.

Hierbei wird erfindungsgemäß das Nährmedium mit einer so großen Menge der Aminosäure, zum Beispiel L-Phenylalanin, versetzt, dass die wässrige Phase noch ungelöste Aminosäure enthält, die sich erst im Verlauf der Reaktion entsprechend der stattfindenden Biokonversion allmählich löst. Da das Substrat nicht toxisch ist, kann es in einer so großen Menge eingesetzt werden, dass ein Substratdepot geschaffen wird, das eine aufwendig geregelte Nachdosierung unnötig macht. Es handelt sich somit um ein sich selbst regulierendes System, in dem genau so viel Aminosäure nachgelöst wird, wie die Mikroorganismen umsetzen. Somit liegt ohne aufwendige technische Maßnahmen ständig eine optimale Substratkonzentration vor.

Da es sich bei der Biokonversion der Aminosäuren zu den Aromastoffen um eine wachstumsgekoppelte Reaktion handelt, wird dem Nährmedium als Kohlenstoffquelle zum Beispiel Glukose, sowie Vitamine und Mineralien nachgefüttert, um nicht- limitiertes Zellwachstum zu gewährleisten. Um über einen möglichst langen Zeitraum eine hohe Biokonversionsrate zu erzielen, kann die Aminosäure auch in fester Form nachdosiert werden, wodurch das Substratdepot ständig aufrecht erhalten bleibt. Es ist kein technischer Aufwand nötig, da keine besondere Dosierrate erforderlich ist, solange der Zustand der Übersättigung erhalten bleibt.

Da die bei dem erfindungsgemäßen Verfahren entstehenden Produkte zu einer Produktinhibierung führen, muss eine Methode zur in situ-Produktabtrennung gewählt werden. Hierfür hat sich ein Zweiphasensystem mit Polypropylenglykol 1.200 als organischem, mit dem Nährmedium nicht mischbarem Extraktionsmittel besonders bewährt, wobei es vorzugsweise im Verhältnis 1 : 1 der Kulturbrühe zugefügt wird. Polypropylenglykol besitzt einen hohen Verteilungskoeffizienten für das aus L-Phenylalanin entstehende 2-Phenylethanol und extrahiert dieses somit aus der wässrigen Phase. Die 2-Phenylethanol-Konzentration im Nährmedium bleibt somit niedrig, so dass es höchstens zu einer ganz geringen Produktinhibierung kommt.

Polypropylenglykol hat den weiteren Vorteil, dass es mit dem wässrigen Medium keine stabilen Emulsionen bildet. Deshalb kann das durch ständiges Rühren während der Reaktion entstandene Gemisch anschließend durch eine Zentrifugation leicht getrennt werden.

Mit dem erfindungsgemäßen Verfahren können zum Beispiel mit Kluyveromyces marxianus CBS 600 im Melassemedium in 25 Stunden 12,5 g/l 2-Phenylethanol und 3 g/l 2-Phenylethylacetat (bezogen auf die wässrige Phase) produziert werden, was einer durchschnittlichen volumetrischen Produktivität von 0,6 g/l pro Stunde entspricht. Mit Saccharomyces cerevisiae DSM 70487 wurden im synthetischen Medium in 47 Stunden 15,5 g/l 2-Phenylethanol (bezogen auf die wässrige Phase) produziert. Dies entspricht einer durchschnittlichen volumetrischen Produktivität von 0,33 g/l pro Stunde.

Damit ist das erfindungsgemäße Verfahren deutlich effizienter als das Verfahren zur extraktiven Biokonversation von 2-Phenylethanol aus Phenylalanin, das in Biotechnol. Prog. 2002, 18, 514 - 523 beschrieben ist. Dort wird Ölsäure als Extraktionsmittel verwendet und dabei eine Gesamtproduktkonzentration von 12,6 g/l erreicht (24 g/l 2-Phenylethanol in der organischen Phase) was einer durchschnittlichen volumetrischen Produktivität von 0,26 g/l 2-Phenylethanol pro Stunde entspricht. Die Raum-Zeit-Ausbeute ist somit beim erfindungsgemäßen Ansatz deutlich größer und verspricht eine höhere Wirtschaftlichkeit des Verfahrens.

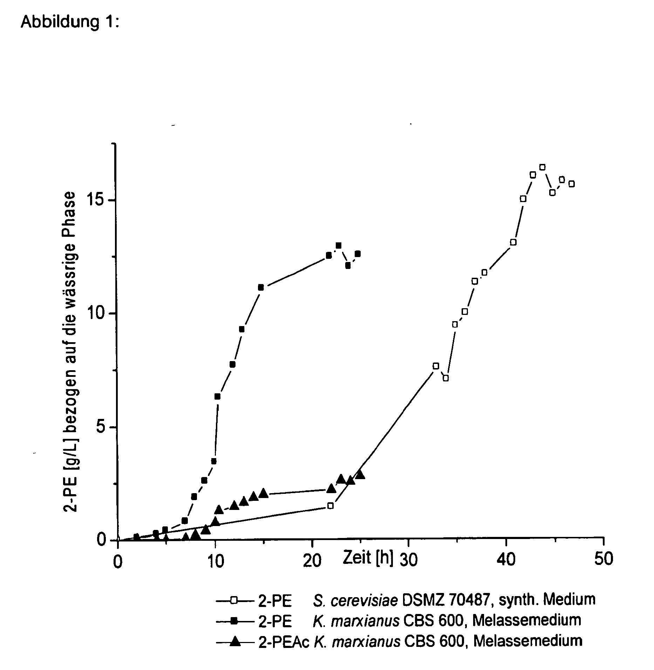

Das erfindungsgemäße Verfahren wird durch die nachfolgenden Beispiele und die Abbildung näher erläutert. Dabei zeigt
- Abb. 1: die Prozesskinetiken von Kluyveromyces marxianus CBS 600 in melassehaltigem Medium und Saccharomyces cerevisiae DSM 70487 in synthetischem Medium, jeweils mit Polypropylenglykol 1.200.

### Beispiel 1

| | |
|---|---|
| Saccharose in Form von Melasse | 8 g/L |
| Glucose | 22 g/L |
| L-Phenylalanin | 40 g/L |
| MgSO₄ | 0,5 g/L |
| KI | 0,0002 g/L |
| Na₂MoO₄ | 0,0004 g/L |
| Biotin | 1,60E-05 g/L |
| Pantothenat | 0,00296 g/L |
| Niacin | 0,0024 g/L |
| Thiamin | 0,0032 g/L |
| p-Aminobenzoesäure | 0,0016 g/L |
| Riboflavin | 0,0016 g/L |
| Inosit | 0,016 g/L |
| Pyridoxin | 0,0032 g/L |
| KH₂PO₄ | 1,24 g/L |
| K₂HPO₄ | 0,16 g/L |

500 ml des Mediums und 500 ml Polypropylenglykol 1200 wurden unter sterilen Bedingungen in einem 2,4 l Bioreaktor KLF 2000 (Bioengineering, Wald, Schweiz) gefüllt und mit einer Übernachtkultur von Kluyveromyces marxianus CBS 600 beimpft (50 ml Melassemedium im 300 ml Erlenmeyerkolben ohne Schikanen, 33°C, 180 rpm bei Auslenkung 25 mm). Der pH-Wert wurde gemessen und durch Zugabe von 1 M NaOH bzw. 1 M H₃PO₄ zwischen 4,5 und 5 gehalten. Es wurde mit einem Volumenstrom von 1 vvm Luft begast, der bei Bedarf bis auf 2,5 vvm erhöht wurde. Die Kultivierungstemperatur betrug 33°C. Die Glucosekonzentration im Medium wurde offline gemessen und bei Bedarf manuell nachdosiert. Eine Nachdosierung des Precursors L-Phenylalanin erübrigte sich, da durch die übersättigte Lösung ein Substratdepot vorlag.

In 25 Stunden konnten so 12,5 g/L 2-Phenylethanol und 3g/L 2-Phenylethylacetat (bezogen auf die wässrige Phase) produziert werden, was einer durchschnittlichen volumetrischen Produktivität von 0,6 g/lh 2-PE + 2-PEAc entspricht.

Die zeitabhängige Produktausbeute wird durch Abb. 1 gezeigt.

### Beispiel 2

| | |
|---|---|
| Glucose | 35 g/L |
| L-Phenylalanin | 40 g/L |
| MgSO₄ | 0,5 g/L |
| KI | 0,0002 g/L |
| Na₂MoO₄ | 0,0004 g/L |
| Biotin | 1,60E-05 g/L |
| Pantothenat | 0,00296 g/L |
| Niacin | 0,0024 g/L |
| Thiamin | 0,0032 g/L |
| p-Aminobenzoesäure | 0,0016 g/L |
| Riboflavin | 0,0016 g/L |
| Inosit | 0,016 g/L |
| Pyridoxin | 0,0032 g/L |
| KH₂PO₄ | 6,2 g/L |
| K₂HPO₄ | 0,8 g/L |

400 ml des Mediums und 400 MI Polypropylenglykol 1200 wurden unter sterilen Bedingungen in einen 2,4 l Bioreaktor KLF 2000 (Bioengineering, Wald, Schweiz) gefüllt und mit einer Übernachtkultur von Saccharomyces cerevisiae DSM 70487 beimpft (50 ml Medium im 300 ml Erlenmeyerkolben ohne Schikanen, 30°C, 180 rpm bei Auslenkung 25 mm). Der pH-Wert wurde gemessen und durch Zugabe von 1 m NaOH bzw. 1m H₃PO₄ zwischen 4,5 und 5 gehalten. Es wurde mit einem Volumenstrom von 1 vvm Luft begast, die Kultivierungstemperatur betrug 30°C. Die Glucosekonzentration im Medium wurde offline gemessen und ab dem Zeitpunkt t=36 Stunden mit einer Rate von 4,8 g/h nachdosiert.

In 47 Stunden wurden so 15,5 g/L 2-Phenylethanol (bezogen auf die wässrige Phase) produziert. Dies entspricht einer durchschnittlichen volumetrischen Produktivität von 0,33 g/lh 2-PE:

Die zeitabhängige Produktausbeute wird durch Abb. 1 gezeigt.

## Patentansprüche

1. Verfahren zur fermentativen Herstellung von 2-Phenylethanol und/oder 2-Phenylethylacetat aus L-Phenylalanin in einem Bioreaktor, wobei man einen Mikroorganismus, der den gewünschten Duft- oder Aromastoff bilden kann, in einem Zweiphasensystem, bestehend aus einem wässrigen Nährmedium und aus einem mit Wasser nicht mischbaren Extraktionsmittel, welches einen hohen Verteilungskoeffizienten für den entstehenden Duft- oder Aromastoff aufweist und ihn in situ aus dem wässrigen Nährmedium entfernt, auf das als Stickstoffquelle dienende L-Phenylalanin einwirken lässt, **dadurch gekennzeichnet, dass** man als Extraktionsmittel Polypropylenglykol mit einem Molekulargewicht > 1000 und L-Phenylalanin in einer seine Löslichkeitsgrenze überschreitenden Menge einsetzt.

2. Verfahren nach Anspruch 1 , **dadurch gekennzeichnet, dass** L-Phenylalanin in einer so großen Menge eingesetzt wird, dass die wässrige Phase noch ungelöstes L-Phenylalanin enthält, das sich erst im Verlauf der Reaktion entsprechend der stattfindenden Biokonversion allmählich löst.

3. Verfahren nach den Ansprüchen 1 bis 2, **dadurch gekennzeichnet, dass** als Mikroorganismen Saccharomyces oder Kluyveromyces-Stämme eingesetzt werden.

4. Verfahren nach den Ansprüchen 1 bis 3, **dadurch gekennzeichnet, dass** es zur gleichzeitigen Herstellung von 2-Phenylethanol und 2-Phenylethylacetat eingesetzt wird.

5. Verfahren nach den Ansprüchen 1 bis 4, **dadurch gekennzeichnet, dass** die für das Zellwachstum erforderliche Kohlenstoffquelle sowie Vitamine und Mineralien in das Nährmedium kontinuierlich nachdosiert werden.

6. Verfahren nach den Ansprüchen 1 bis 5, **dadurch gekennzeichnet, dass** das Nährmedium und das Extraktionsmittel im Verhältnis von etwa 1 : 1 eingesetzt werden.

7. Verfahren nach den Ansprüchen 1 bis 6, **dadurch gekennzeichnet, dass** die im Bioreaktor durch ständiges Rühren entstehende Emulsion durch Zentrifugation getrennt wird.

## Claims

1. Fermentation process for preparing 2-phenyl ethanol and/or 2-phenylethyl acetate from L-phenyl alanine in a bioreactor wherein in a two-phase system comprising an aqueous nutrient medium and an extraction agent which cannot be mixed with water and which has a high distribution coefficient for the resulting perfuming or flavouring agent and removes it in situ from the aqueous nutrient medium, a microorganism which can form the desired perfuming or flavouring agent is allowed to act on the L-phenyl alanine acting as a source of nitrogen, **characterised in that** polypropylene glycol with a molecular weight of > 1000 and L-phenyl alanine in a quantity exceeding its solubility limit are used as the extraction agent.

2. Process in accordance with claim 1 **characterised in that** the L-phenyl alanine is used in such a large quantity that the aqueous phase still contains undissolved L-phenyl alanine which only gradually dissolves during the courses of the reaction corresponding to the bioconversion that is taking place.

3. Process in accordance with claims 1 to 2 **characterised in that** Saccharomyces or Kluyveromyces strains are used as microorganisms.

4. Process in accordance with claims 1 to 3 **characterised in that** it is used for the simultaneous production of 2-phenyl ethanol and 2-phenylethyl acetate.

5. Process in accordance with claims 1 to 4 **characterised in that** the carbon required for cell growth as well as vitamins and minerals are continuously added to the nutrient medium.

6. Process in accordance with claims 1 to 5 **characterised in that** the nutrient medium and the extraction medium are used at a ratio of around 1:1.

7. Process in accordance with claims 1 to 6 **characterised in that** in the emulsion produced in the bioreactor through constant stirring is separated through centrifuging.

## Revendications

1. Procédé de production par fermentation, dans un bioréacteur, de 2-phényléthanol et/ou d'acétate de 2-phényléthyle à partir de la L-phénylalanine, impliquant l'action d'un microorganisme qui est capable de former le parfum ou l'arôme souhaité, sur ladite L-phénylalanine servant de source d'azote, au sein d'un système biphasique constitué d'un milieu de culture aqueux et d'un agent d'extraction non-miscible à l'eau présentant un coefficient de répartition élevé vis-à-vis dudit parfum ou arôme et faisant en sorte que ce dernier soit retiré in situ dudit milieu de culture aqueux, **caractérisé en ce que** l'on met en oeuvre, en tant qu'agent d'extraction, du polypropylèneglycol ayant une masse moléculaire > 1000, la quantité de L-phénylalanine mise en oeuvre étant telle que sa limite de solubilité soit dépassée.

2. Procédé selon la revendication 1, **caractérisé en ce que** la quantité de L-phénylalanine mise en oeuvre est tellement importante que la phase aqueuse contient encore de la L-phénylalanine non-dissoute qui ne se dissoudra qu'au fur et à mesure de la réaction correspondant à une bioconversion progressive.

3. Procédé selon les revendications 1 à 2, **caractérisé en ce que** l'on met en oeuvre, en tant que microorganismes, des souches de Saccharomyces ou de Kluyveromyces.

4. Procédé selon les revendications 1 à 3, **caractérisé en ce qu'**il est mis en oeuvre pour la production simultanée de 2-phényléthanol et d'acétate de 2-phényléthyle.

5. Procédé selon les revendications 1 à 4, **caractérisé en ce que** la source de carbone indispensable pour la croissance cellulaire ainsi que des vitamines et des minéraux sont continuellement rajoutés audit milieu de culture.

6. Procédé selon les revendications 1 à 5, **caractérisé en ce que** ledit milieu de culture et ledit agent d'extraction sont mis en oeuvre dans un rapport d'environ 1 : 1.

7. Procédé selon les revendications 1 à 6, **caractérisé en ce que** l'émulsion qui se forme au sein dudit bioréacteur par une agitation permanente, est séparée par centrifugation.
